(19) Europäisches Patentamt / European Patent Office / Office européen des brevets

(11) **EP 2 077 117 A1**

(12) **EUROPÄISCHE PATENTANMELDUNG**
veröffentlicht nach Art. 153 Abs. 4 EPÜ

(43) Veröffentlichungstag:
**08.07.2009   Patentblatt 2009/28**

(21) Anmeldenummer: **07747912.9**

(22) Anmeldetag: **26.04.2007**

(51) Int Cl.:
*A61K 38/16* (2006.01)          *A61K 39/395* (2006.01)
*A61P 37/02* (2006.01)

(86) Internationale Anmeldenummer:
**PCT/RU2007/000206**

(87) Internationale Veröffentlichungsnummer:
**WO 2007/126336 (08.11.2007 Gazette 2007/45)**

(84) Benannte Vertragsstaaten:
**AT BE BG CH CY CZ DE DK EE ES FI FR GB GR
HU IE IS IT LI LT LU LV MC MT NL PL PT RO SE
SI SK TR**
Benannte Erstreckungsstaaten:
**AL BA HR MK RS**

(30) Priorität: **28.04.2006   RU 2006114457**

(71) Anmelder:
  • **Alikhanov, Khallar Abdumuslimovich
    Moscow, 115573 (RU)**
  • **Kacharava, Leonid Yazonovich
    Tbilisi, 380019 (GE)**

(72) Erfinder:
  • **ALIKHANOV, Bagdadi Abumuslimovich
    Moscow, 111672 (RU)**
  • **PROKOPENKO, Petr Georgievich
    Moscow, 117463 (RU)**
  • **TERENTIEV, Alexandr Alexandrovich
    Moscow, 117292 (RU)**

(74) Vertreter: **Jeck, Anton et al
    Jeck - Fleck - Herrmann
    Patentanwälte
    Klingengasse 2/1
    71665 Vaihingen/Enz (DE)**

(54) **MITTEL MIT IMMUNREGULATORISCHER WIRKUNG UND SEINE ANWENDUNG ZUR BEHANDLUNG VON AUTOIMMUNKRANKHEITEN**

(57)   Die Erfindung bezieht sich auf ein Mittel mit immunregulatorische Eigenschaften und einem beschleunigten klinischen Effekt im Exazerbationsstadium, das vorwiegend bei der Behandlung von Autoimmunkrankheiten eingeht wird und Trophobiast-β-1-Glykoprotein (TBG) und Immunglobulin (Ig) aufweist. Darüber hinaus wird ein Immunglobulin der G-Klasse (Ig-G) oder A-Klasse (Ig-A) oder M-Klasse (Ig-M) eingesetzt.

**Beschreibung**

Die Erfindung betrifft in Mittel nach dem Oberbegriff des Anspruchs 1.

Die Erfindung Mittel für die Medizin, insbesondere neue bioaktive Substanzen (BAS), die über immunregulatorische Eigenschaften verfügen, und ihre Anwendung. Sie können in der praktischen Medizin zur Behandlung von Autoimmunkrankheiten sowie in der Experimentalchemie und in der Tierheilkunde verwendet werden.

Es sind Immunglobuline für intravenöse Injektion bekannt, welche zur Behandlung von Autoimmunkrankheiten eingesetzt werden. (Siehe Pacetti P., Garau D., Caramatti C., Mangoni L., Zamboni V., Canova N., Rizzoli V. Assessment of the efficacy of a last-generation polyvalent immunoglobulin in the treatment of idiopathic thrombocytopenic purpura // Curr. Med. Res. Opin. - 1997. - 13. - No. 9. - P. 517-527).

Das bekannte Mittel ist jedoch bei der Behandlung im Exazerbationsstadium der Autoimmunkrankheit nicht geeignet. Es dient lediglich zum Einsatz während der Remission. Außerdem ist die Einführung von großen Dosen von Immunglobulinen im Laufe von mehreren Jahren erforderlich, um einen klinischen Effekt zu erreichen.

Seinem technischen Wesen nach liegt der angemeldeten Erfindung das Trophoblast-β-1-Glykoprotein (TBG) nahe. Dieses weist immunregulatorische Eigenschaft auf und dient zur Behandlung von unterschiedlichen Autoimmunkrankheiten (Siehe Patent der RF Nr. 2056852, Kl. A 61 K 35/50, 1994).

Jedoch kann das bekannte Mittel seine Wirkung bei seiner Anwendung während der Exazerbation einer Autoimmunkrankheit erst 4 bis 5 Tage nach der Einführung dieses Mittels in den Körper des Kranken entwickeln.

Es ist Aufgabe der Erfindung ein Mittel zu entwickeln, das immunregulatorische Eigenschaft und beschleunigten klinischen Effekt im Exazerbationsstadium vorwiegend bei der Behandlung von Autoimmunkrankheiten aufweist.

Die gestellte Aufgabe wird durch das Merkmal des Anspruchs 1 gelöst.

Das Mittel mit der Möglichkeit die Suppressionsaktivität der mononuklearen Zellen (suppressive activity of mononuclear cells, MNC) sowie die Absonderung von Zytokinen (citokins) durch diese Zellen zu induzieren, enthält das Trophoblast-β-1-Glykoprotein (pregnancy specific glycoprotein, TBG). Erfindungsgemäß enthält dieses Mittel zusätzlich Immunglobulin (Ig). Darüber hinaus wird ein Immunglobulin der G-Klasse (Ig-G) oder A-Klasse (Ig-A) oder M-Klasse (Ig-M) eingesetzt. Dabei werden TBG und Ig-G in gleichen Anteilen bzw. in solchen Anteilen genommen, deren Verhältnis jeweils 1:19 beträgt.

Bei der Behandlung von Autoimmunkrankheiten wird ein Mittel eingesetzt, welches das Vermögen aufweist, die Suppressionsaktivität der mononuklearen Zellen und die Absonderung von Zytokinen durch diese Zellen zu induzieren. Dieses Mittel enthält TBG und Ig. Dabei wird ein Immunglobulin der G-Klasse (Ig-G), oder A-Klasse (Ig-A) oder M-Klasse (Ig-M) eingesetzt. Das TBG und das Ig-G werden in gleichen Anteilen bzw. in solchen Anteilen verwendet, deren Verhältnis jeweils 1:19 beträgt. Darüber hinaus wird das TBG- und Ig-haltige Mittel bei der Behandlung von Autoimmunkrankheiten parenteral eingeführt.

Das Wesen der Erfindung besteht darin, dass das angemeldete Mittel mit bestimmtem TBG- und Ig-Gehalt das Vermögen besitzt, die proliferative Aktivität der mononuklearen Zellen zu unterdrücken und ihre Suppressionsaktivität und die Absonderung von Zytokinen TGF-B1 (transforming growth factor-beta-1), IL-10 (interleukin), IL-6 durch diese Zellen zu induzieren. Darüber hinaus ermöglicht der Einsatz des angemeldeten Mittels einen höheren klinischen Effekt zu erreichen. Dieser Effekt tritt erst am 1. Tag nach seiner Einführung ein.

Das TBG kann aus den Abfallprodukten (A-Fraktion) bei der Herstellung von Gammaglobulin (Urheberschein Nr. 1341736, Kl. A 61 K35/16, 1985) gewonnen werden.

Das Immunglobulin Ig kann nach einem allgemein bekannten Verfahren gewonnen werden (Siehe z. B., V.V. Anastassiev, Immunglobulin für intravenöse Injektion, Nizhny Novgorod, NGMA-Verlag, 2000).

Das angemeldete Mittel kann mittels Vermischung der Ausgangskomponenten TBG und Ig in Verhältnissen 1:99; 1:19; 1:9; 1:1,5; 1:1 im Glasgeschirr bei Temperatur von +1 bis 10 °C gewonnen werden.

Der Heileffekt des angemeldeten Mittels besteht darin, das es das Vermögen aufweist, die Suppressionsaktivität der mononuklearen Zellen der Kranken mit Autoimmunkrankheiten sowie die Absonderung der Zytokinen TGF-B1, IL-10 durch die mononukleare Zellen zu induzieren.

Deswegen kann behauptet werden, dass das angemeldete Mittel eine immunregulatorische Eigenschaft aufweist.

Es sei bemerkt, dass das Mittel mit den oben genannten Komponenten TBG und Ig-G in unterschiedlichen Anteilen bereitet wurde. Jedoch wurden keine wesentlichen Unterschiede in den Versuchsergebnissen bei der Gewinnung des Mittelsmit unterschiedlichen Anteilverhältnissen festgestellt.

Immerhin kann unter Berücksichtigung des wirtschaftlichen Ansatzes das TBG zu Ig-G Verhältnis von 1:19 empfohlen werden.

Dieses Verhältnis gilt als optimal sowohl aus Sicht der Wirksamkeit des Mittels als auch in Hinsicht auf seine Zubereitungsfreundlichkeit. Das gleiche positive Ergebnis wurde bei der Forschung mehrmals auch bei einem Verhältnis von 1:99 erhalten.

Es sei bemerkt, dass als Ig die unterschiedlichen Klassen (Arten) eingesetzt wurden: Ig-G, Ig-A und Ig-M. Es wurden ungefähr die gleichen Ergebnisse erreicht. Jedoch kann in der Praxis die Ig-G Klasse bevorzugt werden, da ihre Herstellung einfacher ist.

Die Bioaktivität des angemeldeten Mittels, bestehend aus TBG und Ig, wurde folgenderweise festgestellt:

Beispiel 1

Es wurde die Wirkung des aus TBG und Ig-G bestehenden Mittels auf die proliferative Aktivität der mononuklearen Zellen des periphen Bluts erforscht. Diese Zellen wurden nach dem Boyum-Verfahren 1969 ausgesondert.

Der Bereich der benutzten Dosen betrug 1 $\mu$g/ml - 960 $\mu$g/ml.

Die Forschungsergebnisse sind in der Tabelle 1 gegeben.

Tabelle 1

| TBG-Anteil (rein), $\mu$g/ml | 1 | 3 | 6 | 15 | 30 | 60 | 120 | 240 | 480 | 960 |
|---|---|---|---|---|---|---|---|---|---|---|
| Inhibitionsindex, % | 0 | 4 | 14 | 23 | 31 | 44 | 37 | 23 | 13 | 0 |
| TBG-Anteil im Mittel mit Ig-G | 0,5 | 1,5 | 3 | 7,5 | 15 | 30 | 60 | 120 | 240 | 480 |
| Inhibitioiisindex, % | 0 | 5 | 15 | 24 | 32 | 46 | 38 | 25 | 14 | 0 |

Aus Tabelle 1 ist ersichtlich, dass das aus TBG und Ig-G bestehende Mittel die durch das Phytohämagglutinin verursachte proliferative Aktivität der mononuklearen Zellen des periphen Bluts um das Zweifache stärker unterdrückt, als das reine TBG.

Beispiel 2

Es wurde der Einfluss des angemeldeten aus TBG und Ig-G bestehenden Mittels auf die Fähigkeit, die Suppressionsaktivität der mononuklearen Zellen des periphen Bluts zu induzieren, erforscht.

In der ersten Stufe wird die mononukleare Zelle nach dem Verfahren der Zellensedimentation beim einstufigen Gradient von Ficoll-Urotrast (Boyum-Methode) erzeugt.

Das periphe Blut wird dem Blutspender durch Venenpunktion in der gleichen Zeit entnommen. Das Blut wird in die Prüfgläser mit Heparinlösung ausgehend von 1 ml Blut: 20 - 30 Einheiten von Heparin gesetzt. Danach wird das Blut mit der Hanks-Lösung ohne Ca$^{++}$ und Mg$^{++}$ im Verhältnis 1:2 verdünnt und auf den Gradient Ficoll-Urotrast aufgeschichtet (Dichte 1,078).

Danach wird das Zentrifugieren im Zustand 400 g innerhalb von 30 Minuten vorgenommen. Die Suspension der mononuklearen Zellen wird aus der Interphase in das Zentrifugenglas übertragen. Es wird die Hanks-Lösung ohne Ca$^{++}$ und Mg$^{++}$ zugegeben. Danach werden 3 aufeinander folgende Zentrifugiervorgänge je 10 Minuten lang vorgenommen, um die Ficoll-Urotrast-Lösung aus den Zellen auszuwaschen. Nach dem 3. Zentrifugiervorgang wird das Sediment der mononuklearen Zellen in 1 ml des 199 Mediums neu aufgeschwemmt, und die Anzahl der mononuklearen Zellen wird anhand der Goryaev-Kammer gezählt.

In der zweiten Stufe werden die mononuklearen Zellen in zwei gleiche Teile aufgeteilt. Der erste Teil davon wird ohne den Aktivator von Suppressoren und der zweite Teil mit dem Aktivator von Suppressoren kultiviert. Als Aktivator wird das erforschte Mittel eingesetzt.

Die mononuklearen Zellen werden in den mit Gummistopfen verschlossenen Penizillinfläschchen Nr. 14,5 bei einer Temperatur von 37 °C kultiviert. Der Kultivierungsnährboden ist RPMI-1640 mit dem Zusatz von 20 % des Serums IV AB der Blutgruppe und Glutamin.

In jedes Fläschchen werden 5 x 10$^6$ Zellen in 2,0 ml des vollen Kultivierungsnährbodens gebracht. Diese Kultur wird zwecks Induktion der Suppressoren mit dem aus TBG und Ig-G bestehenden Mittel in Dosen von 1 - 960 $\mu$g/ml ergänzt.

Die Kultivierung der Zellen erfolgt im Laufe von 48 Stunden. Danach werden die mononuklearen Zellen gewaschen, um den Kultivierungsnährboden zu entfernen. Die Proliferation wird mittels einer Behandlung mit Mitomyzin C 40 $\mu$g/ml innerhalb von 30 Minuten bei 37 °C abgesperrt. Danach erfolgt das dreifache Waschen mit dem Medium 199 mit 5 % Gehalt von Serum IV AB (gekühlt). Das Zellensediment wird neu aufgeschwemmt. Die Anzahl der kernhaltigen Zellen wird ermittelt. Es wird der Prozentanteil der Lebensfähigkeit der Zellen anhand von 0,1 %-ger Trypanblaulösung bestimmt, indem diese Lösung bis zur erforderlichen Konzentration verdünnt wird. Dabei werden alle diese Vorgänge separat an den Vergleichszellen und an der stimulierten Komposition durchgeführt. Zum Auswaschen der Zellen wird silikoniertes Geschirr eingesetzt.

In der nächsten Stufe werden jedem Teil der Vergleichszellen und den durch das Mittel stimulierten mononuklearen Zellen (MNZ) die frisch ausgesonderten MNZ zugegeben. Die letzteren wurden mit Phytohämagglutinin (PHA) stimuliert und dienen als reagierende Test-Zellen in gleichen Anteilen (0,5 x 106 Zellen/ml), um die Test-Kulturen zu bekommen. Die Züchtung erfolgt innerhalb von 72 Stunden. Danach wird die Proliferation der Test-Kulturen anhand von Tidimin H[3] eingeschätzt. Über die Suppressionsgröße wird nach der Proliferationsabnahme geurteilt. Der Suppressionsindex (SI) wird nach folgender Formel ermittelt:

$$SI = \left(1 - \frac{\textit{Anzahl Imp/Min in Testkultur nach Stimul. durchs Mittel}}{\textit{Anzahl Imp/Min in Testkultur ohne Mittel}}\right)100\%.$$

Es wurde der Einfluss des aus TBG und Ig-G bestehenden Mittels auf die Fähigkeit, die Suppressionsaktivität der MNZ zu induzieren, erforscht. Die Ergebnisse dieser Forschung sind in Tabelle 2 enthalten. Dosenbereich 1 - 960 μg/ml.

Tabelle 2

| TBG-Anteil (rein) in μg/ml | 1 | 3 | 6 | 15 | 30 | 60 | 120 | 240 | 480 | 960 |
|---|---|---|---|---|---|---|---|---|---|---|
| Suppr.-Index, % | 0 | 2 | 7 | 16 | 23 | 40 | 26 | 13 | 4 | 0 |
| TBG-Anteil im Mittel mit Ig-G | 0,5 | 1,5 | 3 | 7,5 | 15 | 30 | 60 | 120 | 240 | 480 |
| Suppr.-Index, % | 0 | 3 | 8 | 17 | 24 | 44 | 27 | 15 | 6 | 0 |

Aus der Tabelle 2 ist ersichtlich, dass das aus TBG und Ig-G bestehende Mittel die Suppressionsaktivität der mononuklearen Zellen des periphen Bluts um das Zweifache stärker induziert, als das reine TBG.

Beispiel 3

Es wurde der Einfluss des aus TBG und Ig-G bestehenden Mittels auf die Fähigkeit der mononuklearen Zellen (MNZ) des periphen Bluts, die Produktion der Zytokine - des Transformationswachstumfaktors Beta-1 (TGF - β1) und des Interleukin-10 (IL-10) zu induzieren, erforscht.

In der ersten Stufe wurde die Suspension der mononuklearen Zellen nach dem Verfahren der Zellensedimentation beim einstufigen Gradient von Ficoll-Urotrast (Boyum-Methode) erzeugt.

Die MNZ wurden in 2 gleiche Teile geteilt. Der erste Teil davon wurde ohne das angemeldeten Mittel und der zweite Teil mit dem angemeldeten Mittel kultiviert.

Die MNZ werden in den mit Gummistopfen verschlossenen Penizillin-Fläschchen Nr. 14,5 bei einer Temperatur von 37 °C gezüchtet. Der Kultivierungsnährboden ist RPMI-1640 mit einem Zusatz von 20 % des Serums IV AB der Blutgruppe und Glutamin.

In jedem Fläschchen werden 5 x 10[6] mononukleare Zellen in 1 ml des vollen Kultivierungsnährbodens gezüchtet. Diese Kultur der mononuklearen Zellen wird zwecks Induktion der Gewinnung von Zytokinen TGF-β1 und IL-10 mit dem angemeldeten Mittel ergänzt.

Die Zellenzüchtung erfolgte innerhalb von 24 Stunden. Danach wurde das Kulturmedium von den Zellen mittels Zentrifugierens (1000 g 10 Minuten lang) abgesondert. Dann wurden davon 500 μl entnommen, um die Analyse durchzuführen.

Bei der Bestimmung der Menge von TGF-β1 im Kulturmedium wurde zuerst die Probenextraktion vorgenommen. Diese Phase der Analyse ermöglicht es, die TGF-β1 aus den Komplexen zu befreien und es für die Analyse verfügbar zu machen.

Dafür wurden 0,25 ml (250 μl) des Kulturmediums und 0,05 (50 μl) Extraktionslösung in das Polypropylenprüfglas gesetzt. Der Inhalt wurde im Wirbel (vortex) gemischt. Die Mischung wurde 30 Minuten lang bei 4 °C inkubiert. Danach wurde ins Prüfglas 250 μl Arbeitspufferlösung zur Verdünnung der Standards zugesetzt. In dieser Phase erfolgt die Verdünnung des Kulturmediums um das 2,2-fache.

Um die Analyse durchzuführen, wurde die erforderliche Anzahl von 8 Lochstreifen (8-well strips) aus der zerlegbaren und mit den TGF-β1-Antikörpern bedeckten Mikroplatte genommen.

Um die Zuverlässigkeit der Analyseergebnisse zu erhöhen, wurden die zu untersuchenden und die Vergleichsproben doppelt gesetzt, d. h. für jede Probe wurden zwei Löcher genommen. In die jeweiligen Löcher wurden je 200 μl von jedem Standard und der Extraktionsprobe gesetzt. In alle Löcher wurden je 500 μl biotinylierter TGF-β1-Antikörper zugegeben und mittels Abklopfens der Platte vermischt. Die Platte wurde mit einer Folie abgedeckt und innerhalb von 30 Minuten bei Raumtemperatur inkubiert. Der Inhalt der Löcher wurde vollständig entfernt. Alle Löcher wurden 4-fach mit Arbeitspufferlösung abgewaschen, indem je 400 ml dieser Lösung jedesmal in alle Löcher nachgesetzt wurden. Dabei wurde die Lösung jedesmal vollständig in die Streifenlöcher gefüllt und dann daraus entfernt. Nach dem Durchwaschen wurden die Feuchtigkeitsreste entfernt, indem die umgekehrten Streifen gegen das Filterpapier abgeklopft wurden. Jedes Loch bis auf die Chromogenblindprobe wurde mit 100 $\mu$l der Arbeitslösung von Streptavidin-Peroxidase-Konjugat ergänzt. Die Platte wurde mit einer Folie abgedeckt und 30 Minuten lang bei Raumtemperatur inkubiert. Der Löcherinhalt wurde vollständig entfernt. Die Löcher wurden 4-mal mit Arbeitspufferlösung gewaschen. Danach wurden je 100 $\mu$l der Chromogenlösung von Tetramethylbenzidin (TMB) in alle Löcher hineingebracht. Dann erfolgte die Inkubation im Dunkeln im Laufe von 20 - 30 Minuten bei Raumtemperatur bis die Kalibrierungsprobe mit maximalem TGF-$\beta$1 Gehalt in den Löchern blau gefärbt wurde.

Um die Enzymreaktion zu stoppen, wurden je 100 $\mu$l der Stop-Lösung (1H-Lösung der Schwefelsäure) in jedes Loch hineingebracht. Die Reagenzien wurden mittels vorsichtigen Abklopfens gegen den Streifenhalter vermischt. Die blaue Farbenlösung wurde durch eine gelbe abgelöst.

Die Ergebnisse wurden fotometrisch am Fotometer für immunenzymatische Analyse bei einer Wellenlänge von 450 nm sofort nach dem Aufhören der Enzymreaktion erfasst.

Um die TGF-$\beta$1-Konzentration in den zu erforschenden Proben zu bestimmen, wurde eine Kalibrationskurve in folgenden Koordinaten konstruiert: X-Achse = Konzentration von TGF-$\beta$1 in Kalibrationsproben (rg/ml); Y-Achse = entsprechender Wert der optischen Dichte.

Anhand der Kalibrationskurve und ausgehend vom resultierenden Wert der optischen Dichte wurde die Konzentration von TGF-$\beta$1 in den erforschten Proben festgestellt. Sind die Proben vorher verdünnt worden, so wurden die resultierenden Konzentrationswerte mit dem Verdünnungsfaktor (10,100,1000 usw.) multipliziert.

Um die IL-10-Menge im Kulturmedium einzuschätzen, wurde die für die Durchführung erforderliche Menge der 8 Lochstreifen aus der zerlegbaren und mit den IL-10-Antikörpern bedeckten Mikroplatte genommen.

Um die Zuverlässigkeit der Analyseergebnisse zu erhöhen, wurden die zu untersuchenden und die Vergleichsproben doppelt gesetzt, indem für jede Probe zwei Löcher benutzt wurden.

In die jeweiligen Löcher wurden je 50 $\mu$l von jedem Standard, der Prüf- und der Vergleichsprobe gesetzt. Es wurden je 50 $\mu$l des Inkubationspuffers für die Löcher mit den Standards und je 50 $\mu$l der Verdünnungsarbeitslösung in die Löcher mit dem Kulturmedium addiert.

Die Platte wurde mit einer Folie abgedeckt und innerhalb von 2 Stunden bei Raumtemperatur inkubiert.

Der Löcherinhalt wurde vollständig entfernt.

Alle vier Löcher wurden vierfach mit Arbeitspufferlösung gewaschen, indem sie jedesmal mit je 400 $\mu$l in allen Löchern ergänzt wurde. Dabei wurde die Lösung jedesmal vollständig in die Streifenlöcher gefüllt und dann daraus entfernt. IL-10 in allen Löchern wurde mit je 100 $\mu$l der biotinylierten Antikörper ergänzt und vermischt, indem die Platte abgeklopft wurde.

Die Platte wurde mit einer Folie abgedeckt und innerhalb von 2 Stunden bei Raumtemperatur inkubiert. Der Löcherinhalt wurde vollständig entfernt. Alle Löcher wurden 4-mal mit Arbeitspufferlösung gewaschen. Danach wurden je 100 $\mu$l der Arbeitslösung von Streptavidin-Peroxidase-Konjugat in jedes Loch bis auf Chromogenblindprobe zugegeben.

Die Platte wurde mit einer Folie abgedeckt und 30 Minuten lang bei Raumtemperatur inkubiert. Der Löcherinhalt wurde vollständig entfernt. Die Löcher wurden 4-mal mit Arbeitspufferlösung gewaschen.

Danach wurden je 100 $\mu$l der Chromogenlösung von Tetramethylbenzidin (TMB) in alle Löcher hineingebracht.

Dann erfolgte die Inkubation im Dunkeln im Laufe von 20 - 30 Minuten bei Raumtemperatur bis die Kalibrierungsprobe mit maximalem IL-10 Gehalt in den Löchern blau gefärbt wurde.

Um die Enzymreaktion zu stoppen, wurden je 100 $\mu$l der Stop-Lösung (1H-Lösung der Schwefelsäure) in jedes Loch hineingebracht. Die Reagenzien wurden mittels vorsichtigen Abklopfens gegen den Streifenhalter vermischt. Die blaue Farbenlösung wurde durch die gelbe abgelöst.

Die Erfassung der Ergebnisse wurde fotometrisch am Fotometer für immunenzymatische Analyse bei einer Wellenlänge von 450 nm sofort nach dem Aufhören der Enzymreaktion vorgenommen.

Um die IL-10-Konzentration in den zu erforschenden Proben zu bestimmen, wurde eine Kalibrationskurve mit folgenden Koordinaten konstruiert: X-Achse - Konzentration von IL-10 in Kalibrationsproben (rg/ml); Y-Achse - entsprechender Wert der optischen Dichte.

Anhand der Kalibrationskurve und ausgehend vom resultierenden Wert der optischen Dichte wurde die Konzentration von IL-10 in den erforschten Proben festgestellt. Sind die Proben vorher verdünnt worden, so müssen die resultierenden Konzentrationswerte mit dem Verdünnungsfaktor (10, 100, 1000 usw.) multipliziert werden.

Infolge der durchgeführten Forschungen wurde festgestellt, dass das TBG- und Ig-G-haltige Mittel über das Vermögen verfügt, die Produktion von gleichzeitig 2 Zytokinen - TGF-$\beta$1 und IL-10 - durch die mononuklearen Zellen des periphen Bluts zu induzieren.

Dabei ist der Spiegel der durch die mononuklearen Zellen des periphen Bluts unter Wirkung des angemeldeten Mittels erzeugten IL-10 und TGF-β1 Zytokine 2 bis 3-fach so hoch wie unter der Wirkung von reinen TBG. Die Verabreichung des angemeldeten Mittels einem Kranken schafft die Exazerbation der Autoimmunkrankheiten ab. Die Wirkung wird klinisch bereits am ersten Tag der Behandlung ermittelt, während bei der Behandlung mit reinem TBG lässt sich die Wirkung beim Kranken erst am 4. bis 5. Tag der Behandlung erkennen.

Beispiel 4

Es wurde die Wirkung des aus TBG und Ig-G bestehenden Mittels im Verhältnis von TBG zu Ig-G von 1:99 auf die proliferative Aktivität der mononuklearen Zellen des periphen Bluts erforscht. Diese Zellen wurden nach dem Boyum-Verfahren 1969 ausgesondert.
Der Bereich der benutzten Dosen betrug 1 μg/ml - 960 μg/ml.
Die Forschungsergebnisse sind in der Tabelle 3 angegeben.

Tabelle 3

| TBG-Anteil (rein), μg/ml | 1 | 3 | 6 | 15 | 30 | 60 | 120 | 240 | 480 | 960 |
|---|---|---|---|---|---|---|---|---|---|---|
| Inhibitionsindex, % | 0 | 4 | 14 | 23 | 31 | 44 | 37 | 23 | 13 | 0 |
| TBG-Anteil im Mittel mit Ig-G | 0,5 | 1,5 | 3 | 7,5 | 15 | 30 | 60 | 120 | 240 | 480 |
| Inhibitionsindex, % | 0 | 3 | 13 | 24 | 33 | 45 | 37 | 24 | 14 | 0 |

Aus der Tabelle 3 ist ersichtlich, dass das aus TBG und Ig-G bestehende Mittel im Verhältnis 1:99 die proliferative Aktivität der mononuklearen Zellen des periphen Bluts unterdrückt. Diese proliferative Aktivität wird durch das Phytohämagglutinin verursacht. Die Unterdrückung ist ebenso stark wie beim Einsatz des angemeldeten Mittels mit TBG und Ig-G-Gehalt im Verhältnis von 1:19.

Beispiel 5

Es wurde die Wirkung des aus TBG und Ig-G im Verhältnis von TBG zu Ig-G von 1:1,5 bestehenden Mittels auf die proliferative Aktivität der mononuklearen Zellen des periphen Bluts erforscht. Diese Zellen wurden nach dem Boyum-Verfahren 1969 ausgesondert.
Der Bereich der benutzten Dosen betrug 1 μg/ml - 960 μg/ml.
Die Forschungsergebnisse sind in der Tabelle 4 gegeben.

Tabelle 4

| TAG-Antcil (rein), μg/ml | 1 | 3 | 6 | 15 | 30 | 60 | 120 | 240 | 480 | 960 |
|---|---|---|---|---|---|---|---|---|---|---|
| Inhibitionsindex, % | 0 | 4 | 14 | 23 | 31 | 44 | 37 | 23 | 13 | 0 |
| TBG-Anteil im Mittel mit Ig-G | 0,5 | 1,5 | 3 | 7,5 | 15 | 30 | 60 | 120 | 240 | 480 |
| Inhibitionsindex, % | 0 | 4,5 | 13 | 22 | 33 | 45 | 37,5 | 24 | 15 | 0 |

Aus der Tabelle 4 ist ersichtlich, dass das aus TBG und Ig-G im Verhältnis 1:1,5 bestehende Mittel die proliferative Aktivität der mononuklearen Zellen des periphen Bluts unterdrückt. Die proliferative Aktivität wird durch das Phytohämagglutinin verursacht. Der Unterdrückungsgrad ist ebenso hoch wie beim Einsatz des angemeldeten Mittels mit TBG und Ig-G-Gehalt im Verhältnis von 1:19 und 1:99.

Beispiel 6

Es wurde die Wirkung des aus TBG und Ig-G im Verhältnis von TBG zu Ig-G von 1:9 bestehenden Mittels auf die proliferative Aktivität der mononuklearen Zellen des periphen Bluts erforscht. Diese Zellen wurden nach dem Boyum-Verfahren 1969 ausgesondert.
Der Bereich der benutzten Dosen betrug 1 μg/ml - 960 μg/ml.
Die Forschungsergebnisse sind in der Tabelle 5 gegeben.

Tabelle 5

| TBG-Anteil (rein), μg/ml | 1 | 3 | 6 | 15 | 30 | 60 | 120 | 240 | 480 | 960 |
|---|---|---|---|---|---|---|---|---|---|---|
| Inhibitionsindex, % | 0 | 4 | 14 | 23 | 31 | 44 | 37 | 23 | 13 | 0 |
| TBG-Anteil im Mittel mit Ig-G | 0,5 | 1,5 | 3 | 7,5 | 15 | 30 | 60 | 120 | 240 | 480 |
| Inhibitionsindex, % | 0 | 3,5 | 13 | 22 | 29 | 42 | 36 | 26 | 14 | 0 |

Aus Tabelle 5 ist ersichtlich, dass das aus TBG und Ig-G im Verhältnis 1:9 bestehende Mittel die proliferative Aktivität der mononuklearen Zellen des periphen Bluts unterdrückt. Die proliferative Aktivität wird durch das Phytohämagglutinin verursacht. Der Unterdrückungsgrad ist ebenso hoch wie beim Einsatz des angemeldeten Mittels mit TBG und Ig-G-Gehalt im Verhältnis von 1:19, 1:99 und 1:1,5.

Beispiel 7

Es wurde die Wirkung des aus TBG und Ig-G im Verhältnis von TBG zu Ig-G von 1:1 bestehenden Mittels auf die proliferative Aktivität der mononuklearen Zellen des periphen Bluts erforscht. Diese Zellen wurden nach dem Boyum-Verfahren 1969 ausgesondert.
Der Bereich der benutzten Dosen betrug 1 μg/ml - 960 μg/ml.
Die Forschungsergebnisse sind in der Tabelle 6 gegeben.

Tabelle 6

| TBG-Anteil (rein), μg/ml | 1 | 3 | 6 | 15 | 30 | 60 | 120 | 240 | 480 | 960 |
|---|---|---|---|---|---|---|---|---|---|---|
| Inhibitionsindex, % | 0 | 4 | 14 | 23 | 31 | 44 | 37 | 23 | 13 | 0 |
| TBG-Anteil im Mittel mit Ig-G | 0,5 | 1,5 | 3 | 7,5 | 15 | 30 | 60 | 120 | 240 | 480 |
| Inhibitionsindex, % | 0 | 5 | 11 | 24 | 29 | 45 | 36 | 22 | 14 | 0 |

Aus Tabelle 6 ist ersichtlich, dass das aus TBG und Ig-G im Verhältnis 1:9 bestehende Mittel die proliferative Aktivität der mononuklearen Zellen des periphen Bluts unterdrückt. Die proliferative Aktivität wird durch das Phytohämagglutinin verursacht. Der Unterdrückungsgrad ist ebenso hoch wie beim Einsatz des angemeldeten Mittels mit TBG und Ig-G-Gehalt im Verhältnis von 1:19, 1:99, 1:9 und 1:1,5.

Beispiel 8

Die Forschungsergebnisse im Zusammenhang mit dem Einfluss des aus TBG und Ig-G im Verhältnis 1:99 bestehenden Mittels auf das Vermögen, die Suppressionsaktivität der MNZ zu induzieren, sind in Tabelle 7 enthalten.
Der Dosenbereich beträgt 1 - 960 μg/ml.

Tabelle 7

| TBG-Anteil (rein), μg/ml | 1 | 3 | 6 | 15 | 30 | 60 | 120 | 240 | 480 | 960 |
|---|---|---|---|---|---|---|---|---|---|---|
| Suppr.-Index, % | 0 | 2 | 7 | 16 | 23 | 40 | 26 | 13 | 4 | 0 |
| TBG-Anteil im Mittel mit Ig-G | 0,5 | 1,5 | 3 | 7,5 | 15 | 30 | 60 | 120 | 240 | 480 |
| Suppr.-Tndex, % | 0 | 3,5 | 8 | 18 | 24 | 38 | 28 | 17 | 6 | 0 |

Aus der Tabelle 7 ist ersichtlich, dass das aus TBG und Ig-G im Verhältnis von 1:99 bestehende Mittel die Suppressionsaktivität der mononuklearen Zellen des periphen Bluts im gleichen Maße induziert, wie das Mittel mit TBG und Ig-G-Gehalt im Verhältnis von 1:19. Dieses Mittel kann auch die Produktion der Zytokine des Transformationswachstumfaktors Beta-1 TGF-$\beta$1 und Interleukin-10 (IL-10) durch die mononuklearen Zellen (MNZ) des periphen Bluts induzieren.

Beispiel 9

Die Forschungsergebnisse im Zusammenhang mit dem Einfluss des aus TBG und Ig-G im Verhältnis 1:9 bestehenden Mittels auf das Vermögen, die Suppressionsaktivität der MNZ zu induzieren, sind in Tabelle 8 enthalten.

Der Dosenbereich beträgt 1 - 960 μg/ml.

Tabelle 8

| TBG-Antcil (rein), μg/ml | 1 | 3 | 6 | 15 | 30 | 60 | 120 | 240 | 480 | 960 |
|---|---|---|---|---|---|---|---|---|---|---|
| Suppr.-Index, % | 0 | 2 | 7 | 16 | 23 | 40 | 26 | 13 | 4 | 0 |
| TBG-Anteil im Mittel mit Ig-G | 0,5 | 1,5 | 3 | 7,5 | 15 | 30 | 60 | 120 | 240 | 480 |
| Suppr-.Index, % | 0 | 5 | 15 | 25 | 33 | 46 | 35 | 22 | 14 | 0 |

Aus der Tabelle 8 ist ersichtlich, dass das aus TBG und Ig-G im Verhältnis von 1:9 bestehende Mittel die Suppressionsaktivität der mononuklearen Zellen des periphen Bluts im gleichen Maße induziert, wie das Mittel mit TBG und Ig-G-Gehalt im Verhältnis von 1:19 und 1:99. Dieses Mittel kann auch die Produktion der Zytokine des Transformationswachstumfaktors Beta-1 TGF-β1 und Interleukin-10 (IL-10) durch die mononuklearen Zellen (MNZ) des periphen Bluts induzieren.

Beispiel 10

Die Forschungsergebnisse im Zusammenhang mit dem Einfluss des aus TBG und Ig-G im Verhältnis 1:1,5 bestehenden Mittels auf das Vermögen, die Suppressionsaktivität der MNZ zu induzieren, sind in Tabelle 9 enthalten.

Der Dosenbereich beträgt 1 - 960 μg/ml.

Tabelle 9

| TBG-Anteil (rein), μg/ml | 1 | 3 | 6 | 15 | 30 | 60 | 120 | 240 | 480 | 960 |
|---|---|---|---|---|---|---|---|---|---|---|
| Suppr.-Index, % | 0 | 2 | 7 | 16 | 23 | 40 | 26 | 13 | 4 | 0 |
| TBG-Anteil im Mittel mit Ig-G | 0,5 | 1,5 | 3 | 7,5 | 15 | 30 | 60 | 120 | 240 | 480 |
| Suppr.-Index, % | 0 | 3,5 | 11 | 25 | 33 | 47 | 35 | 21 | 15 | 0 |

Aus der Tabelle 9 ist ersichtlich, dass das aus TBG und Ig-G im Verhältnis von 1:1,5 bestehende Mittel die Suppressionsaktivität der mononuklearen Zellen des periphen Bluts im gleichen Maße induziert, wie das Mittel mit TBG und Ig-G-Gehalt im Verhältnis von 1:19, 1:99 und 1:9. Dieses Mittel kann auch die Produktion der Zytokine des Transformationswachstumfaktors Beta-1 TGF-β1 und Interleukin-10 (IL-10) durch die mononuklearen Zellen (MNZ) des periphen Bluts induzieren.

Beispiel 11

Die Forschungsergebnisse im Zusammenhang mit dem Einfluss des aus TBG und Ig-G im Verhältnis 1:1 bestehenden Mittels auf das Vermögen, die Suppressionsaktivität der MNZ zu induzieren, sind in Tabelle 10 enthalten.

Der Dosenbereich beträgt 1 - 960 μg/ml.

Tabelle 10

| TBG-Anteil (rein), μg/ml | 1 | 3 | 6 | 15 | 30 | 60 | 120 | 240 | 480 | 960 |
|---|---|---|---|---|---|---|---|---|---|---|
| Suppr.-Index, % | 0 | 2 | 7 | 16 | 23 | 40 | 26 | 13 | 4 | 0 |
| TRG-Anteil im Mittel mit Ig-G | 0,5 | 1,5 | 3 | 7,5 | 15 | 30 | 60 | 120 | 240 | 480 |
| Suppr.-Index, % | 0 | 4 | 9 | 22 | 29 | 42 | 33 | 19 | 8 | 0 |

Aus der Tabelle 10 ist ersichtlich, dass das aus TBG und Ig-G im Verhältnis 1:1 bestehende Mittel die Suppressionsaktivität der mononuklearen Zellen des periphen Bluts im gleichen Maße induziert, wie das Mittel mit TBG und Ig-G-Gehalt im Verhältnis von 1:19, 1:99, 1:9 und 1:1,5. Dieses Mittel kann auch die Produktion der Zytokine des Transformationswachstumfaktors Beta-1 TGF-β1 und Interleukin-10 (IL-10) durch die mononuklearen Zellen (MNZ) des periphen Bluts induzieren.

Beispiel 12 - Auszug aus einer Krankengeschichte

Kranker O. 43 J. befand sich in ärztlicher Behandlung in der 5. therapeutischen Abteilung mit folgender Diagnose: Seropositive progredient chronische Polyarthritis mit Systemerscheinungen (Fieber, Lymphadenopathie, Anämie, Amyotrophien, Neuropathie, rheumatoide Knötchen), Aktivität des III. Grades, das III. Stadium, Insuffizienz der Gelenkfunktion III.

Bei der Krankenhauseinlieferung: Beschwerden über ausgeprägte Schmerzen in kleinen und großen Gelenkextremitäten (proximale Interphalangealhände, Fingergrund-, proximale Hand-, Ellenbogen-, Schulter-, Knie-, obere Sprung-, Mittelfußphalangeal-, innere Schlüsselbeingelenke), ihre Anschwellung, Bewegungseinschränkung, ausgeprägte Steifheit der Gelenke innerhalb des ganzen Tages, Ameisenlaufenempfindung an den Hand- und Fußspitzen, Fieber bis zu 38,6 Grad.

Arthralgien wurden im Laufe von 7 Jahren empfunden. Seit 5 Jahren gibt es mäßige Gelenkschmerzen und Gelenkanschwellungen. Nichtsteroide Antiphlogistika wurden unregelmäßig genommen. Um die Schmerzen zu mildern, genoss der Kranke öfters Alkohol. Die tatsächliche Rekrudeszenz erfolgte 2 Wochen vor der stationären Aufnahme, nämlich nach der akuten respiratorischen Infektion: Temperaturerhöhung bis zu febrilen Werten, vielfache Gelenkanschwellungen. Die oben genannten Beschwerden ließen sich stärker fühlen. Bei ambulanter Therapie erhielt der Kranke Antibiotika und nichtsteroide Antiphlogistika. In den letzten Tagen begann er, Alkohol einzunehmen. Danach erfolgte die Krankenhauseinweisung.

Bei der Krankenhauseinweisung wurde ein schwerer Zustand festgestellt. Körpertemperatur 38,6 Grad. Komplette Bewegungslähmung wegen Gelenkschmerzen und -anschwellungen. Der Kranke stöhnte vor Schmerzen. Blasse Hautdecken. Mäßige Schwellungen der Unterschenkel. Das Abtasten ergab vergrößerte (1,5 x 1,5 cm, 1,5 x 2 cm) Lymphknoten (Submandibularlymphknoten, axiallare und Leistenknoten). Verschärftes Atmen in den Lungen (Raucher). Herztöne waren mäßig gedämpft. Tachykardie bis zu 92 pro Minute, regelmäßiger Puls, mit zufriedenstellender Periode, ABD 130/80 mm Hg. Weicher Bauch, unauffällig (schmerzlos). Leber + 2 cm. Die Milz wird nicht tastbar. Negatives Pasternazki-Merkmal.

Ausgeprägte Extremitätenamyotrophien. Ausgeprägte Anschwellung, Entformung der Gelenke, und zwar der der proximalen Interphalangealhände, Fingergrund-, proximalen Hand-, Ellenbogen-, Knie- und oberen Sprunggelenke. Hyperthermie der Haut über den Gelenken. Begrenzung von aktiven und passiven Gelenkbewegungen. Ulnare Deviation der Hände. Kontrakturen der proximalen Hand- und Ellenbogengelenke. Rheumatoides Knötchen im Bereich des Ellenbogengelenks links.

Röntgenaufnahme der Hände: Osteoporose, Verschmälerung der Gelenkspalte, zahlreiche Erosionen, unvollständige Verrenkungen.

Blutanalyse: Hämoglobin - 86 g/l, L-11200, Blutsenkungsgeschwindigkeit - 67 mm/St., CRP (C-reaktives Protein) -4+, Rheumafaktor - 1:320, HBs-(-), Asparagintransaminase (AnSAT)-74, Alanine aminotransferase (ALAT)-56, Gesamteiweißmenge - 66g/l.

Suppressionsaktivität der T-Lymphozyte des periphen Bluts bei der Induktion des angemeldeten Mittels betrug 16 %.

Sinusrhythmus der EKG, diffuse Myokardänderungen. Nach der Röntgenografie der Thoraxorgane wurden keine Herd- und infiltrative Änderungen festgestellt.

Behandlung des Kranken: Reopyrin täglich à 3 ml intramuskulär, Diclophenac dreimal täglich à 50 mg, Injektionen von Analginum mit Dimedrolum zweimal täglich.

Der Zustand des Kranken blieb ohne wesentliche Dynamik innerhalb von 10 Tagen. Anämie, Fieber bis zu 37,9 - 38,8, ausgeprägte exsudative Gelenkänderungen blieben erhalten. Der Kranke war bewegungslos.

Dem Kranken wurde die Behandlung in Form von täglichen intravenösen Injektionen des angemeldeten TBG und Ig-G-haltigen Mittels mit den Anteilen von 1:99 vorgeschlagen.

Der Zustand des Kranken wies wesentliche Verbesserung auf. Die Schmerzen in allen Gelenken und ihre Anschwellungen ließen merklich nach. Die Beweglichkeit nahm zu. Die Körpertemperatur wurde normalisiert. Die Gelenksteifheit verschwand. Danach konnte der Kranke selbst gehen und sich bedienen. Der Hämoglobinspiegel stieg bis auf 125 g/l an. Die Blutsenkungsgeschwindigkeit nahm bis auf 27 mm/St. ab. CRP 1+, Gesamteiweißmenge wurde bis 84 g/l erhöht.

Während der erneuten Analyse wurde die Steigerung der Suppressionsaktivität der T-Lymphozyte des periphen Bluts bei der Induktion des angemeldeten Mittels bis 64,2 % festgestellt. Im Zusammenhang mit der Ouchterlony-Reaktion wurden beim Kranken am 7., 14. und 28. Tag keine Antikörper gegen das angemeldete Mittel erkannt.

Der Kranke wurde aus dem Krankenhaus im zufriedenstellenden Zustand entlassen.

Es wurde dem Kranken empfohlen, die Einnahme der nichtsteroiden Antiphlogistika (Diclophenac) ambulatorisch fortzusetzen und Basispräparate (Sulfasalazine) zu verordnen.

Beispiel 13.

Kranker M., 35 J., befand sich in ärztlicher Behandlung mit folgender Diagnose: Seropositive progredient chronische Polyarthritis mit Systemerscheinungen (Amyotrophien, Anämie, Neuropathie, Fieber) Aktivität des II. Grades, das II. Stadium, Insuffizienz der Gelenkfunktion II.

Bei der Krankenhauseinlieferung: Beschwerden über Schmerzen in proximalen Interphalangealgelenken, Fingergrundgelenken der Hände, proximalen Handgelenken, Schulter-, Knie-, oberen Sprunggelenken; Anschwellungen der genannten Gelenke, Bewegungseinschränkung in diesen Gelenken. Beschwerden über ausgeprägte Morgensteifheit der Gelenke, die bis zu Mittag dauerte; Ameisenlaufen an Finger- und Zehspitzen, subferile Körpertemperatur.

Der Patient ist seiner Meinung nach seit 5 Jahren krank. Die Krankheit begann mit Schädigung der oberen Sprung- und Kniegelenke. In den letzten 2 Jahren kam die Anschwellung der proximalen Handgelenke und der Interphalangealgelenke der Hände dazu. Ab dieser Zeit bemerkte er auch die Morgensteifheit der Gelenke. Es wurde die Behandlung mit nichtsteroiden Antiphlogistika mit positivem Effekt vorgenommen. Die tatsächliche Verschlimmerung begann vor 3 Wochen. Nach der akuten Respirationskrankheit entwickelte sich die ausgeprägte Anschwellung der proximalen Interphalangealgelenke, der Fingergrundgelenke, der proximalen Hand-, Knie- und der oberen Sprunggelenke. Die Morgensteifheit wurde schlimmer (sie dauerte bis Mittag) und die Körpertemperatur stieg bis 37,5 Grad. Die durchgeführte Behandlung mit nichtsteroiden Antiphlogistika und Antibiotika (Klacid) ergab keine wesentliche Wirkung. Deswegen erfolgte die Krankenhauseinweisung.

Der Kranke war bei der Krankenhauseinweisung bewegungslos, konnte sich nur mit Mühe bewegen und verwendete einen Stock.

Entformung, Anschwellung der proximalen Interphalangealgelenke und der Fingergrundgelenke der Hände, der proximalen Hand-, Knie-, oberen Sprunggelenke. Begrenzung der aktiven und der passiven Gelenkbewegungen. Hyperthermie der Knie-, proximalen Hand- und oberen Sprunggelenke. Kontrakturen der proximalen Handgelenke. Ausgeprägte Amyotrophien der Schenkel- und Schultermuskeln. Es wurden keine ausgeprägten Pathologien der Organe festgestellt.

Blutanalyse: He-110 g/l, L.-10700, Blutsenkungsgeschwindigkeit -45 mm/St., CRP 3+, Rheumafaktor 1:160, Gesamteiweißmenge 70 g/l, Asparagintransaminase (An-SAT)-40, Alanine aminotransferase (ALAT)-34. Harnanalyse: spezifisches Gewicht 1025, L-1-2 im Blickfeld.

Röntgenografie der Handgelenke: Osteoporose, Verschmälerung der Gelenkspalte, zahlreiche Erosionen.

Vorgenommene Behandlung des Kranken: nichtsteroide Antiphlogistika (Diclophenacinjektionen, Reopyrininjektionen), Magnet- und Lasertherapie, physikalische Therapie.

Der Zustand des Kranken hatte keine ausgeprägte positive Dynamik. Subfebrile Temperatur, Gelenkanschwellungen blieben erhalten, die Gelenksteifheit nahm wesentlich zu. Die Blutsenkungsgeschwindigkeit erhöhte sich bis zu 48 mm/St.ac, CRP 3+.

Die Suppressionsaktivität der T-Lymphozyte des periphen Bluts bei der Induktion des angemeldeten Mittels mit TBG und Ig-G-Gehalt betrug 20 %.

Es wurden tägliche Injektionen des angemeldeten Mittels mit TBG und Ig-G-Gehalt (1:19) verordnet.

Der Zustand des Kranken verbesserte sich wesentlich. Die Gelenksteifheit verschwand. Die Körpertemperatur normalisierte sich. Die Gelenkschmerzen und ihre Anschwellung nahmen wesentlich ab. Die Anzahl der möglichen Bewegungen wurde vergrößert. Der Kranke konnte sich frei in den Räumen des Krankenhauses bewegen, er wurde aktiv. Die Druckkraft der Hände wurde von 5 mm Hg bis auf 90 mm Hg (linke Hand) und bis auf 110 mm Hg (rechte Hand) erhöht. Die Anschwellungen der oberen Sprung- und Kniegelenke verschwanden. Der Hämoglobinspiegel im Blut nahm bis zu 140 g/l zu. Die Blutsenkungsgeschwindigkeit wurde bis auf 22 mm/St. reduziert. CRP 1+.

Während der erneuten Analyse wurde die Steigerung der Suppressionsaktivität der T-Lymphozyte des periphen Bluts bei der Induktion des angemeldeten Mittels bis 60,4 % festgestellt. Im Zusammenhang mit der Ouchterlony-Reaktion wurden beim Kranken am 7., 14. und 28. Tag keine Antikörper gegen das angemeldete Mittel erkannt.

Der Kranke wurde aus dem Krankenhaus im zufriedenstellenden Zustand zwecks ambulatorischer Behandlung entlassen.

Um die Sicherheit des aus TBG und Ig-G bestehenden angemeldeten Mittels zu studieren, bevor es klinisch angewendet wird, ist seine akuten Giftigkeit erforscht worden. Das Studium der akute Giftigkeit wurde in Übereinstimmung mit Methodischen Empfehlungen des Pharmakologischen Komitees der RF "Anforderungen an das vorklinische Studium der gesamttoxischen Wirkung der neuen pharmakologischen Stoffe", Moskau, 2001, durchgeführt. Die Forschungsergebnisse zeugen davon, dass sich bei der intraabdominalen Einführung der 1000-fachen Dosis des angemeldeten Mittels keine toxische Wirkung erkennen ließ, und dass bei diesen Dosen es unmöglich war, sein LD50 zu erreichen. Somit ist das vorgeschlagene aus TBG und Ig-G bestehende Mittel nicht giftig.

Obwohl die oben angeführten Erfindungen ziemlich eingehend und detailliert beschrieben sind, liegt es einem Fachmann auf diesem Gebiet unter Berücksichtigung der Offenbarung dieser Erfindungen nahe, dass weitere bestimmte Änderungen und Modifikationen der Erfindungen vorgenommen werden können, ohne daß vom Grundgedanken bzw.

dem Anwendungsbereich der vorgeschlagenen Ansprüchen abgegangen wird.

Die hier dargelegten Vorteile des vorgeschlagenen Mittels mit TBG- und Ig-Gehalt stellen die Möglichkeit seiner breiten Anwendung sowohl in wissenschaftlichen Forschungen als auch in der praktischen Medizin, Veterinärmedizin sowie in der experimenteller Biochemie sicher.

**Patentansprüche**

1. Mittel mit der Möglichkeit die Suppressionsaktivität der mononuklearen Zellen und die Absonderung von Zytokinen durch diese Zellen zu induzieren, das einen Trophoblast-β-1-Glykoprotein (TBG)-Gehalt aufweist,
   **dadurch gekennzeichnet,**
   **dass** es zusätzlich Immunglobulin (Ig) enthält.

2. Mittel nach Anspruch 1,
   **dadurch gekennzeichnet,**
   **dass** ein Immunglobulin der G-Klasse (Ig-G) oder A-Klasse (Ig-A) oder M-Klasse (Ig-M) eingesetzt wird.

3. Mittel nach Anspruch 1,
   **dadurch gekennzeichnet,**
   **dass** TBG und Ig in gleichen Anteilen verwendet wird.

4. Mittel nach Anspruch 1,
   **dadurch gekennzeichnet,**
   **dass** TBG und Ig im Verhältnis 1:19 zueinander verwendet wird.

5. Anwendung des Mittels mit der Möglichkeit, die Suppressionsaktivität der mononuklearen Zellen und die Absonderung von Zytokinen durch diese Zellen zu induzieren und das TBG und Immunglobulin (Ig) enthält um Autoimmunkrankheiten zu behandeln.

6. Anwendung des Mittels nach Anspruch 5,
   **dadurch gekennzeichnet,**
   **dass** ein Immunglobulin der G-Klasse (Ig-G) oder A-Klasse (Ig-A) oder M-Klasse (Ig-M) eingesetzt wird.

7. Anwendung des Mittels nach Anspruch 6,
   **dadurch gekennzeichnet,**
   **dass** TBG und Ig in gleichen Anteilen verwendet werden.

8. Anwendung des Mittels nach Anspruch 6,
   **dadurch gekennzeichnet,**
   **dass** TBG und Ig im Verhältnis 1:19 zueinander verwendet werden.

9. Anwendung des Mittels nach Anspruch 5,
   **dadurch gekennzeichnet,**
   **dass** dieses Mittel parenteral eingeführt wird.

## INTERNATIONAL SEARCH REPORT

International application No.

PCT/RU2007/0000206

| A. CLASSIFICATION OF SUBJECT MATTER | |
|---|---|
| According to International Patent Classification (IPC) or to both national classification and IPC | *A61K 38/16* *(2006.01)*<br>*A61K 39/395* *(2006.01)*<br>*A61P 37/02* *(2006.01)* |

**B. FIELDS SEARCHED**

Minimum documentation searched (classification system followed by classification symbols)
A61 K 38/16, 38/39, 39/395, A61 P 37/02

Documentation searched other than minimum documentation to the extent that such documents are included in the fields searched

Electronic data base consulted during the international search (name of data base and, where practicable, search terms used)

Esp@cenet, PubMed, DERWENT

**C. DOCUMENTS CONSIDERED TO BE RELEVANT**

| Category* | Citation of document, with indication, where appropriate, of the relevant passages | Relevant to claim No. |
|---|---|---|
| A | RU 2056852 C I (GOLOVISTIKOV IVAN NIKOLAEVICH) 27.03.1996 | 1-9 |
| A | RU 2001 101454 A (STATENS SERUM INSTITUT) 20.01.2003 | 1-9 |
| A | US 6150326 A (KACHARAVA LEONID YAZONOVICH) 21.1 1.2000 | 1-9 |
| A | V.G. GALAKTIONOV. Evoljutsya, IKTS "Akademkniga", 2005, Moscow, 2005, 62-63 | 1-9 |
| A | SU 1836957 AI (2-Y MOSKOVSKY MEDITSINSKY INSTITUT IM. I. I. PIROGOVA) 30.08.1993 | 1-9 |

☐ Further documents are listed in the continuation of Box C.　　☐ See patent family annex.

| | |
|---|---|
| *　Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 10 August 2007 (10.08.2007) | 23 August 2007 (23.08.2007) |

| Name and mailing address of the ISA/ | Authorized officer |
|---|---|
| | |
| Facsimile No. | Telephone No. |

Form PCT/ISA/210 (second sheet) (July 1998)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2056852 A

- WO 1341736 A

**In der Beschreibung aufgeführte Nicht-Patentliteratur**

- **Pacetti P. ; Garau D. ; Caramatti C. ; Mangoni L. ; Zamboni V. ; Canova N. ; Rizzoli V.** Assessment of the efficacy of a last-generation polyvalent immunoglobulin in the treatment of idiopathic thrombocytopenic purpura. *Curr. Med. Res. Opin.,* 1997, vol. 13 (9), 517-527

- Immunglobulin für intravenöse Injektion. **V.V. Anastassiev.** Nizhny Novgorod. NGMA-Verlag, 2000
- *Anforderungen an das vorklinische Studium der gesamttoxischen Wirkung der neuen pharmakologischen Stoffe,* 2001